# FASCICULE DE BREVET EUROPEEN

(11) **EP 4 045 096 B1**
(45) Date de publication et mention de la délivrance du brevet: **14.05.2025**
(21) Numéro de dépôt: 20768637.9
(22) Date de dépôt: 16.09.2020
(51) Int. Cl.: A61L 2/08

(54) **PROCEDE DE DECONTAMINATION DE RECIPIENTS ET DE BOUCHONS ET UNITE DE DECONTAMINATION CORRESPONDANTE**
VERFAHREN ZUR DEKONTAMINATION VON BEHÄLTERN UND KAPPEN UND ENTSPRECHENDE DEKONTAMINATIONSEINHEIT
METHOD FOR DECONTAMINATING CONTAINERS AND CAPS AND CORRESPONDING DECONTAMINATION UNIT

(30) Priorité: 16.09.2019 FR 1910175
(43) Date de publication de la demande: 24.08.2022
(73) Titulaire: Sidel Participations, 76930 Octeville-sur-Mer (FR)
(72) Inventeur: LE PECHOUR, Anthony, 76930 OCTEVILLE-SUR-MER (FR); CHOMEL, Nicolas, 76930 OCTEVILLE-SUR-MER (FR)
(74) Mandataire: Sidel Group
(86) Numéro de dépôt international: PCT/EP2020/075852
(87) Numéro de publication internationale: WO 2021/053007

(56) Documents cités:
- JP-A- H 111 212
- US-A1- 2007 237 672

## Description

Le domaine de l'invention est celui de la conception et de la fabrication de récipients en matière plastique.

Plus précisément, l'invention concerne une unité de décontamination pour une machine de fabrication de récipients en matière plastique.

Lors de la fabrication des récipients en matière plastique, des préformes sont chauffées puis soufflées pour obtenir une forme définitive ou quasiment définitive.

Lorsque le récipient est dans sa forme définitive, il est rempli puis bouché avant d'être mis en pack ou palettisé et expédié chez les distributeurs.

Préalablement à leur remplissage et leur bouchage, les récipients sont décontaminés de sorte que toute ou partie des microorganismes présents dans la bouteille, notamment lors du soufflage, soit éliminée et que le contenu du récipient, une fois rempli, ne puisse être dégradé, notamment dans ses qualités organoleptiques.

Pour cela, les récipients, une fois dans leur forme définitive, traversent une unité de décontamination dans laquelle ils sont décontaminés, par exemple en étant déplacés devant un rayonnement de décontamination prenant la forme d'un faisceau d'électrons qui élimine toute ou partie des microorganismes.

En parallèle de cela, des bouchons, qui peuvent également être en contact avec le produit contenu dans les récipients, sont également décontaminés et traversent une deuxième unité de décontamination, distante de la première unité de décontamination, puis sont dirigés vers une bouchonneuse qui permet de fixer les bouchons sur les récipients après remplissage de ces derniers.

Ainsi, les bouchons et les récipients suivent chacun un cheminement différent pour être décontaminés, et sont réunis pour l'étape de bouchage.

Il en résulte donc une consommation électrique importante de la machine de fabrication de récipients en matière plastique.

Pour pallier cela, il est possible d'utiliser une seule unité de décontamination dans laquelle, dans un premier temps, des récipients sont tout d'abord décontaminés avant d'être stockés en vue de leur remplissage, puis ensuite les bouchons sont à leur tour décontaminés en vue d'être stockés pour l'étape de bouchonnage.

Cela implique cependant que les récipients et les bouchons une fois décontaminés doivent être stockés dans un environnement stérile.

Ainsi, il est nécessaire que la machine de fabrication dispose de lieux de stockage des bouchons et des récipients décontaminés et de zones de convoyage stériles pour assurer le déplacement des bouchons et des bouteilles depuis l'unité de décontamination jusqu'à leur lieu de stockage.

En effet, si les bouchons ou les récipients sont stockés à l'air libre avant d'être utilisés, ou sont convoyés à l'air libre, ils peuvent de nouveau être contaminés par des microorganismes présents dans l'air ambiant par exemple. JP H11 1212 A, US 2007/237672 A1 décrivent des unités pour la décontamination des bouchons et des récipients.

L'invention a notamment pour objectif de pallier les inconvénients de l'art antérieur. Plus précisément, l'invention a pour objectif de proposer une unité de décontamination pour une machine de fabrication de récipients en matière plastique, qui soit à la moins coûteuse et moins encombrante comparée aux unités de l'art antérieur.

L'invention a également pour objectif de fournir une telle unité de décontamination qui permette de réduire le temps de cycle de la fabrication de récipients en matière plastique depuis l'obtention du récipient jusqu'à son bouchage hermétique par un bouchon.

L'invention a en outre pour objectif de fournir une telle unité de décontamination qui soit simple de maintenance.

Ces objectifs, ainsi que d'autres qui apparaîtront par la suite, sont atteints grâce à l'invention.

Selon un aspect, l'invention porte sur un procédé de décontamination d'une série de bouchons et d'une série de récipients en matière plastique destinés chacun à être obturés par un des bouchons (6) de la série de bouchons, le procédé comprenant les étapes suivantes :
- émettre un rayonnement de décontamination par les moyens d'émission,
- convoyer la série de récipients par des premiers moyens de préhension vers les moyens d'émission selon un chemin de convoyage et
- convoyer la série de bouchons par des deuxièmes moyens de préhension vers les moyens d'émission selon le chemin de convoyage ; procédé dans lequel, lors du convoyage de la série de bouchons et de la série de récipients, les deuxièmes moyens de préhension sont en alternance le long du chemin de convoyage avec les premiers moyens de préhension devant les moyens d'émission.

Avantageusement, les récipients et bouchons présentent un axe et dans lequel les récipients et/ou les bouchons sont mis en rotation au tour de l'axe au moins pendant l'étape d'émission du rayonnement de décontamination.

Avantageusement, le rayonnement de décontamination comprend un faisceau d'électrons.

Selon un autre aspect, l'invention porte sur une unité de décontamination pour la mise en œuvre du procédé précité, comprenant :
- des moyens d'émission d'un rayonnement de décontamination,
- des premiers moyens de convoyage présentant des premiers moyens de préhension, et les convoyer vers les moyens d'émission;
- des deuxièmes moyens de convoyage présentant des deuxièmes moyens de préhension pour maintenir les bouchons et les convoyer vers les moyens d'émission, dans lequel deux premiers moyens de préhension contigus définissent entre eux un espacement (E), caractérisée en ce que un des deuxièmes moyens de convoyage est disposés dans l'espacement entre les deux premiers moyens de convoyage contiguës en présentant une portion de convoyage dans laquelle les deuxièmes moyens de préhension sont en alternance avec les premiers moyens de préhension devant les moyens d'émission.

Selon une variante, l'invention porte également sur une unité de décontamination pour machine de fabrication de récipients en matière plastique destinés à être obturés par des bouchons, l'unité de décontamination comprenant :
- des moyens d'émission d'un rayonnement de décontamination,
- des premiers moyens de convoyage présentant des premiers moyens de préhension pour maintenir des récipients par leur col, et les convoyer vers les moyens d'émission ;

caractérisée en ce que l'unité de décontamination comprend également des deuxièmes moyens de convoyage présentant des deuxièmes moyens de préhension pour maintenir les bouchons, et les convoyer vers les moyens d'émission,
et en ce que deux premiers moyens de préhension contigus définissent entre eux un espacement, les deuxièmes moyens de convoyage étant disposés par rapport aux premiers moyens de convoyage en présentant une portion de convoyage dans laquelle les deuxièmes moyens de préhension sont en alternance avec les premiers moyens de préhension devant les moyens d'émission.

Une telle unité de décontamination permet ainsi de réaliser une décontamination simultanée des bouchons et des récipients.

Ainsi, l'encombrement de la machine de fabrication de récipients est réduit, tout comme sa consommation électrique notamment.

De plus, une telle unité de décontamination permet de limiter la quantité de rayonnement de décontamination inutilisé, notamment entre les cols des récipients puisque les bouchons sont intercalés entre ces derniers. Une partie du rayonnement de décontamination initialement perdu est donc ici utilisée.

Avantageusement, les premiers moyens de préhension et les deuxièmes moyens de préhension sont situés dans un même plan.

Cela permet, par exemple, de conserver l'encombrement d'une unité de décontamination tout en augmentant sa capacité d'action pour la décontamination simultanée des bouchons et des récipients.

De préférence, l'unité de décontamination comprend :
- un carrousel d'entrée communicant avec une première ligne d'alimentation en récipients et une deuxième ligne d'alimentation en bouchons ;
- un carrousel de traitement alimenté par le carrousel d'entrée ;
- un carrousel de sortie alimenté par le carrousel de traitement,
chacun du carrousel d'entrée, du carrousel de traitement et du carrousel de sortie portant les premiers moyens de convoyage et les deuxièmes moyens de convoyage.

Cette configuration de l'unité de décontamination permet de faciliter le convoyage des récipients et des bouchons. En effet, il n'est pas nécessaire de multiplier le nombre de carrousels puisqu'un même carrousel peut convoyer à la fois les bouchons et les récipients.

Selon un mode préféré de réalisation, les moyens de préhension comprennent des moyens d'entraînement en rotation des récipients et des bouchons sur le carrousel de traitement.

Les moyens d'entraînement en rotation des récipients et des bouchons sur le carrousel de traitement permettent que l'intégralité de la surface des corps et des bouchons soit traitée par le rayonnement de décontamination lors de son exposition.

En pivotant, les bouchons et récipients sont ainsi totalement exposés au rayonnement de décontamination, et donc entièrement décontaminés.

Dans ce cas, les moyens d'entraînement en rotation comprennent avantageusement :
- un tampon destiné à venir au contact des récipients et des bouchons pour les entraîner en rotation par friction ;
- des moyens moteurs pour mettre en rotation les tampons.

L'entraînement des bouchons et des récipients par contact avec le tampon permet d'éviter de les endommager lors de leur rotation. En effet, un système comprenant des pinces peut laisser des marques d'enfoncement sur les bouchons et les récipients.

De préférence, les tampons sont mobiles entre :
- une position d'utilisation dans laquelle ils sont au contact des récipients ou des bouchons ;
- une position de repos dans laquelle ils sont écartés des récipients ou des bouchons,
chaque tampon étant positionné dans sa position d'utilisation ou dans sa position de repos par un chemin de came et un galet solidaire du tampon.

L'entraînement des tampons entre leur position d'utilisation et de repos est ainsi piloté mécaniquement si bien qu'il n'engendre pas de commande dédiée consommatrice d'énergie.

Par ailleurs un pilotage mécanique du mouvement des tampons présente une grande durée de vie dans le temps et un faible risque d'usure.

Avantageusement, les premiers moyens de préhension comprennent des moyens de support des récipients sur le carrousel de traitement.

Ces socles permettent d'augmenter la bonne tenue des récipients dans l'unité de décontamination.

Selon un mode de réalisation préféré, les moyens de support comprennent des socles destinés à recevoir un fond des récipients, chaque socle étant mobile entre :
- une position d'utilisation dans laquelle il est au contact du fond des récipients ;
- une position de repos dans laquelle il est écarté du fond des récipients, chaque socle étant positionné dans sa position d'utilisation ou dans sa position de repos par un chemin de came et un galet solidaire du socle.

L'entraînement des socles entre leur position d'utilisation et de repos est ainsi piloté mécaniquement si bien qu'il n'engendre pas de commande dédiée consommatrice d'énergie.

Par ailleurs un pilotage mécanique du mouvement des socles présente une grande durée de vie dans le temps et un faible risque d'usure.

Avantageusement, chaque socle est libre en rotation dans sa position d'utilisation.

Ainsi, les socles accompagnent le mouvement de rotation des récipients, ce qui empêche tout frottement pouvant détériorer le fond des récipients.

Avantageusement, le rayonnement de décontamination comprend un faisceau d'électrons.

Avantageusement, le premier moyen de convoyage est adapté pour coopérer avec la bague de support du col du récipient. Le bouchon ne présente pas de baque de support de sorte que les premiers moyens de préhension sont bien disctincts des deuxièmes moyens de préhension.

L'invention concerne également une machine de fabrication de récipients, comprenant :
- une unité de soufflage ;
- une unité de bouchage ;
- une unité de décontamination telle que précédemment décrite,
l'unité de décontamination étant intercalée entre l'unité de soufflage et l'unité de bouchage.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
[Fig. 1] est une vue schématique en perspective d'une machine de fabrication de récipients en matière plastique, comprenant une unité de décontamination selon l'invention ;
[Fig. 2] La figure 2 est une vue en coupe longitudinale de dessus illustrant une unité de décontamination selon l'invention ;
[Fig. 3] La figure 3 est une vue schématique représentant un alignement de récipients et de bouchons tels qu'ils sont destinés à être positionnés dans l'unité de décontamination selon l'invention ;
[Fig. 4] La figure 4 est une vue de détails selon l'encadré de la figure 2 ;
[Fig. 5] La figure 5 est une vue de détails de moyens d'entraînement en rotation des bouchons et des récipients dans l'unité de décontamination selon l'invention ;
[Fig. 6] est une vue de détails de moyens de support des récipients dans l'unité de décontamination selon l'invention.

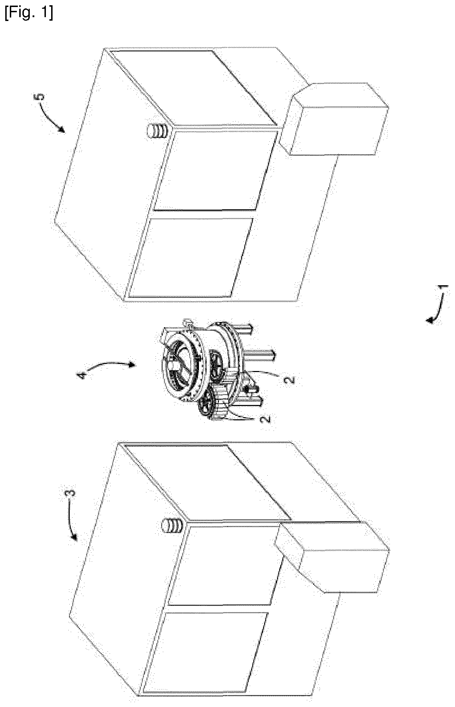

La figure 1 illustre une machine de fabrication 1 de récipients 2 en matière plastique selon l'invention.

Une telle machine de fabrication 1 comprend :
- une unité de soufflage 3 ;
- une unité de décontamination 4 ;
- une unité de bouchage 5.

L'unité de soufflage 3, classiquement connue, permet, à partir de préformes en matière plastique préalablement chauffée, d'obtenir des récipients 2 ayant une forme définitive.

Lorsque les récipients 2 sortent de l'unité de soufflage 3, ils sont alors dirigés vers l'unité de décontamination 4, de sorte à être décontaminés, c'est-à-dire éliminer toute ou partie des microorganismes qu'ils pourraient contenir, préalablement à leur remplissage généralement par un liquide.

Des bouchons 6, destinés à obturer les récipients 2, et généralement fabriqués par injection, sont également décontaminés préalablement à leur utilisation pour le bouchage des récipients 2 dans l'unité de bouchage 5, lorsque les récipients 2 sont remplis.

L'unité de bouchage 5 est alimentée d'une part par des récipients 2 remplis selon une première ligne d'alimentation 7, et d'autre part par des bouchons 6 décontaminés, selon une deuxième ligne d'alimentation 8.

Dans l'unité de bouchage 5, les bouchons 6 sont alors mis en place sur les récipients 2 remplis, notamment sur leur col, de sorte à les fermer hermétiquement et ainsi y confiner le produit qu'ils contiennent, généralement un liquide.

Selon les techniques de l'art antérieur, les bouchons 6 et les récipients 2 sont décontaminés chacun de leur côté avant d'être réunis dans l'unité de bouchage 5.

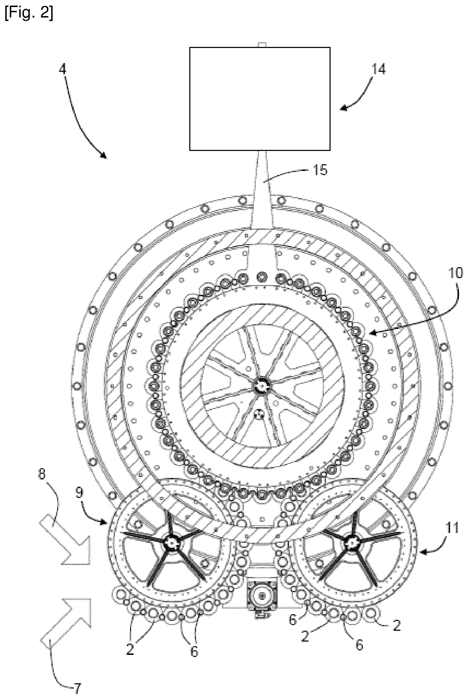

La figure 2 illustre l'unité de décontamination 4 selon l'invention, dans laquelle les bouchons 6 et les récipients 2 sont décontaminés simultanément.

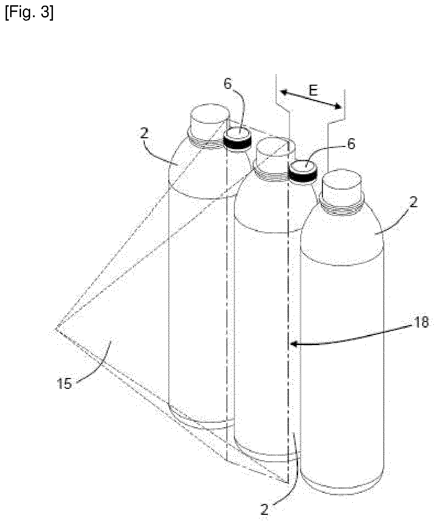

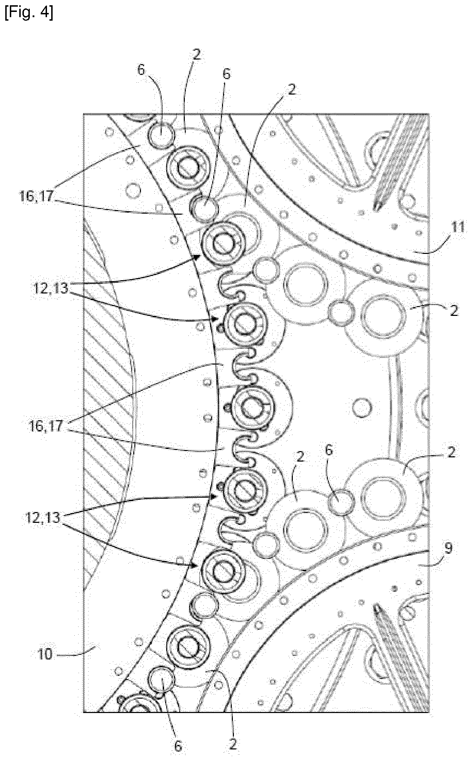

Cela est notamment réalisé par le positionnement astucieux des bouchons 6 et des récipients 2 dans l'unité de décontamination 4, tel qu'illustré sur les figures 3 et 4.

Plus précisément, tel qu'illustré sur les figures 3 et 4, les récipients 2 accolés et sont déplacés en ligne les uns derrière les autres dans l'unité de décontamination 4, et définissent entre eux un espacement E inoccupé.

Plus précisément, entre deux cols successifs de récipients 2, l'espacement E inoccupé permet de loger un bouchon 6 à décontaminer, tel qu'illustré sur les figures 2, 3 et 4.

Ainsi, tel qu'illustré sur la figure 2, l'unité de décontamination 4 est reliée à la première ligne d'alimentation 7 en récipients 2 provenant de l'unité de soufflage 3 ou d'une zone de stockage tampon, et à la deuxième ligne d'alimentation 8 en bouchons 6 provenant d'une zone de stockage ou d'une trémie d'alimentation.

En référence aux figures 2 et 4, l'unité de décontamination 4 comprend :
- un carrousel d'entrée 9 communiquant avec la première ligne d'alimentation 7 en récipients 2 et la deuxième ligne d'alimentation 8 en bouchons 6 ;
- un carrousel de traitement 10 communiquant avec le carrousel d'entrée 9 ;
- un carrousel de sortie 11 communiquant avec le carrousel de traitement 10.

Le carrousel d'entrée 9 permet de recevoir de la part de chacune de la première ligne d'alimentation 7 et de la deuxième ligne d'alimentation 8 des bouchons 6 et des récipients 2 à décontaminer, notamment le col de ces récipients 2.

Par l'alimentation de chacune des lignes, il est ainsi possible, au carrousel d'entrée 9, d'intercaler entre deux cols de récipients 2 à décontaminer, un bouchon 6 à décontaminer.

Pour cela, l'unité de décontamination 4 comprend :
- des premiers moyens de convoyage 12 présentant des premiers moyens de préhension 13 pour maintenir les récipients 2 par leur col et les convoyer vers des moyens d'émission 14 d'un rayonnement de décontamination 15 de l'unité de décontamination 4 ;
- des deuxièmes moyens de convoyage 16 présentant des deuxièmes moyens de préhension 17 des bouchons 6 pour les convoyer vers les moyens d'émission 14.

Tel qu'illustré sur la figure 3 et sur la vue de détail de la figure 4, l'unité de décontamination 4 est configurée de sorte que les premiers moyens de préhension 13 contigus définissent entre eux un espacement E et que les deuxièmes moyens de convoyage 16 sont disposés par rapport aux premiers moyens de convoyage 12 pour positionner les deuxièmes moyens de préhension 17 en alternance avec les premiers moyens de préhension 13 devant les moyens d'émission 14.

Autrement dit, sur chacun des carrousels, il est possible d'observer une alternance entre les premiers moyens de préhension 13 et les deuxièmes moyens de préhension 17. La figure 4 illustre cette alternance en prenant pour exemple le carrousel de traitement 10.

De préférence, les premiers moyens de préhension 13 et les deuxièmes moyens de préhension 17 se présentent sous la forme d'une pince, dont les deux mors sont immobiles l'un par rapport à l'autre. Dans un autre mode de réalisation, les deuxièmes moyens de préhension se présentent sous la forme d'encoches.

En vue de dessus, les moyens de préhension 13, 17 prennent donc la forme d'un U. Tel qu'on le voit sur la figure 3, l'émission du rayonnement de décontamination 15 est réalisée sur une fenêtre 18 sensiblement rectangulaire illustrée en traits mixtes.

Il peut ainsi être constaté que sur la totalité de sa largeur, chaque récipient 2 est atteint par le rayonnement de décontamination 15. Ainsi, au niveau du col qui présente des dimensions inférieures au corps du récipient 2 (notamment en termes de diamètre), le rayonnement de décontamination 15 balaye à la fois le récipient 2 mais également une zone dépourvue de matière de part et d'autre du col.

Ainsi, en intercalant entre deux cols successifs un bouchon 6, le rayonnement de décontamination 15 rencontre alors la matière du bouchon 6, permettant ainsi une décontamination des bouchons 6 simultanément à la décontamination des récipients 2.

Avantageusement, les premiers moyens de préhension 13 et les deuxièmes moyens de préhension 17 sont situés dans un même plan. Les bouchons 6 sont alors situés dans le même plan que les cols des récipients 2.

Selon une première forme de réalisation, les moyens de préhension 13, 17 portés par le carrousel de traitement 10 sont positionnés à un niveau inférieur par rapport aux moyens de préhension 13, 17 portés par le carrousel d'entrée 9 et le carrousel de sortie 11.

Selon une deuxième forme de réalisation, les moyens de préhension 13, 17 portés par le carrousel de traitement 10 sont positionnés à un niveau supérieur par rapport aux moyens de préhension 13, 17 portés par le carrousel d'entrée 10 et le carrousel de sortie 11.

Indépendamment de la première ou de la deuxième forme de réalisation, les moyens de préhension 13, 17 du carrousel de sortie 11 et les moyens de préhension 13, 17 du carrousel d'entrée 10 sont préférentiellement coplanaires.

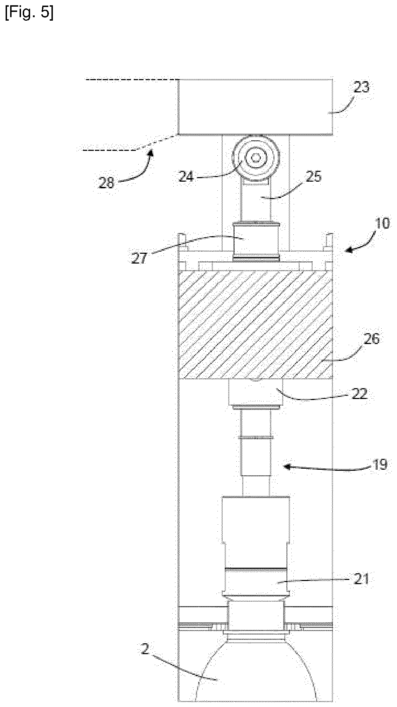

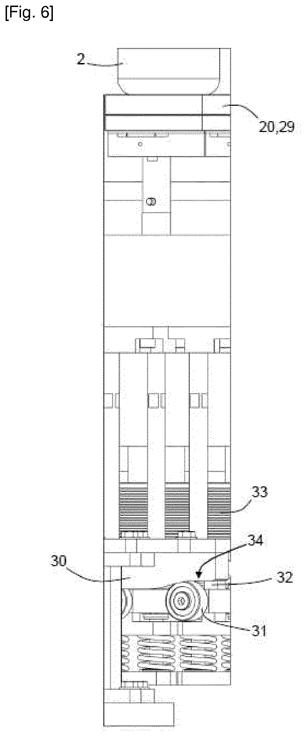

Pour obtenir une décontamination totale des récipients 2 et des bouchons 6, les récipients 2 et les bouchons 6 doivent être tournés de 180°, de sorte à ce que l'intégralité de leur périphérie soit exposée au rayonnement de décontamination 15. Cela est notamment réalisé grâce à des moyens d'entraînement en rotation 19 et à des moyens de support 20 illustrés sur les figures 5 et 6.

En référence à la figure 5, les moyens d'entraînement en rotation 19 des bouchons 6 et des récipients 2 sont à présent décrits.

Ces moyens d'entraînement en rotation 19 comprennent :
- un tampon 21 destiné à venir au contact des récipients 2 et des bouchons 6 pour les entraîner en rotation par friction ;
- des moyens moteurs 22 pour mettre en rotation les tampons 21.

Avantageusement, les tampons 21 sont réalisés dans un matériau élastiquement déformable tel que du caoutchouc, ou dans un matériau inerte ne réagissant pas au rayonnement de décontamination 15.

Ce matériau permet de résister au rayonnement de décontamination 15 d'une part, mais également de modifier sa forme au contact des récipients 2 ou des bouchons 6 pour permettre une coopération par friction entraînant la rotation des bouchons 6 et des récipients 2.

Lors de la prise en charge des récipients 2 et des bouchons 6 par le carrousel de traitement 10, les moyens d'entraînement en rotation 19 doivent être inopérants pour permettre la charge ou la décharge des bouchons 6 et des récipients 2 dans ou hors du carrousel de traitement 10.

Pour cela, les tampons 21 peuvent alors adopter :
- une position d'utilisation dans laquelle ils sont au contact des récipients 2 ou des bouchons 6 ;
- une position de repos dans laquelle ils sont écartés des récipients 2 ou des bouchons 6.

Pour pouvoir adopter sa position d'utilisation ou de repos, chaque tampon 21 est ainsi piloté en déplacement au moyen d'un chemin de came 23 et d'un galet 24 solidaire du tampon 21, le galet 24 coopérant avec le chemin de came 23.

Plus précisément, le chemin de came 23 se présente sous la forme d'une couronne montée au-dessus du carrousel de traitement 10, et le galet 24 est relié au tampon 21 par une tige 25.

La tige 25 traverse alors un bâti 26 solidaire du carrousel de traitement 10, et est montée par le biais de moyens de rappel 27 sur ledit bâti 26. Les moyens de rappel 27 permettent de contraindre le galet 24 à suivre le chemin de came 23.

La couronne formant le chemin de came 23 présente ainsi une surface ayant un bateau 28 permettant de positionner le galet 24 à un point éloigné du carrousel de traitement 10.

Ce bateau forme alors une portion dans laquelle le tampon 21 est dans sa position de repos et donc écarté des bouchons 6 et des récipients 2.

En revanche, lorsque les tampons 21 sont dans leur position d'utilisation, ils sont entraînés en rotation par les moyens moteurs 22 et, par friction, entraînent les bouchons 6 et les récipients 2 en rotation afin de les faire pivoter durant leur passage devant le rayonnement de décontamination 15.

Pour guider les récipients 2 lors de leur rotation dans l'unité de décontamination 4, les premiers moyens de préhension 13 de cette dernière comprend, comme décrit précédemment, des moyens de support 20.

Les moyens de support 20 sont positionnés en regard des moyens d'entraînement en rotation 19 des récipients 2 et sont destinés à recevoir le fond des récipients 2.

Plus précisément, les moyens de support 20 comprennent un socle 29 destiné à venir coopérer avec le fond des récipients 2 pour empêcher leur désaxement lors de leur rotation, et ainsi contraindre les récipients à s'étendre verticalement entre les moyens d'entraînement en rotation 19 et les moyens de support 20.

Tout comme les moyens d'entraînement en rotation 19 qui doivent être écartés des récipients 2 et des bouchons 6 lors des différents échanges entre le carrousel de traitement 10 et le carrousel d'entrée 9 ou le carrousel de sortie 11, les moyens de support 20 doivent également l'être.

Pour cela, le socle 29 peut adopter :
- une position d'utilisation dans laquelle il est au contact du fond des récipients 2 ;
- une position de repos dans laquelle il est écarté du fond des récipients 2.

Les socles 29 sont entraînés chacun dans leur position d'utilisation ou dans leur position de repos par un chemin de came 30 et un galet 31 solidaire du socle.

Plus précisément, chaque galet 31est solidarisée à un socle 29 par une tige 32 montée à translation sur le bâti 26 du carrousel d'entraînement 10.

Le chemin de came 30 se présente alors également sous la forme d'une couronne sur laquelle les galets 31 sont destinés à circuler.

La tige 32 est montée au travers de moyens de rappel 33 sur le bâti du carrousel de traitement 10, les moyens de rappel 33 ayant pour but de maintenir au contact du chemin de came 30 les galets 31 des moyens de support 20.

Le chemin de came 30 présente également un bateau 34 permettant d'écarter le galet 31 du carrousel de traitement 10, notamment dans la position de repos des socles 29, pour permettre les échanges de récipients 2 entre le carrousel 10 de traitement et le carrousel d'entrée 9 ou le carrousel de sortie 11.

Durant la rotation des récipients, les socles 29 sont libres en rotation, ce qui permet d'éviter un frottement des récipients sur les socles 29.

En d'autres termes, les socles 29 suivent le mouvement de rotation des récipients 2 avec lesquels ils coopèrent.

En fonctionnement de l'unité de décontamination 4, c'est-à-dire lors d'un cycle de production, les récipients 2 et les bouchons 6 sont acheminés vers l'unité de décontamination 4 par la première ligne d'alimentation 7 et la deuxième ligne d'alimentation 8 respectivement.

Des moyens de blocage des récipients 2 et des bouchons 6 dans leurs lignes d'alimentation respectives permettent d'autoriser le passage des bouchons 6 ou des récipients 2 vers le carrousel d'entrée 9, de sorte à positionner un bouchon 6 entre deux récipients 2 consécutifs.

Les récipients 2 et les bouchons 6 sont alors entraînés vers l'intérieur de l'unité de décontamination 4 par le carrousel d'entrée 9, jusqu'à atteindre le carrousel de traitement 10, les récipients et les bouchons quittant alors le carrousel d'entrée 9.

Plus précisément, les récipients 2 et les bouchons 6 viennent se loger respectivement dans les premiers moyens de préhension 13 et les deuxièmes moyens de préhension 17 du carrousel d'entraînement 10.

Le carrousel d'entraînement 10 est alors entraîné en rotation de sorte à exposer les récipients 2 et les bouchons 6 au rayonnement de décontamination 15.

Durant la rotation du carrousel de traitement 10, les moyens d'entraînement en rotation 19 et les moyens de support 20 viennent au contact de chacun des récipients 2 pour permettre leur rotation, notamment lorsque ceux-ci sont exposés au rayonnement de décontamination 15.

Les bouchons 6 sont également mis en rotation par application des tampons 21 des moyens de mise en rotation 19 contre ces derniers, les bouchons 6 glissant, lors de leur rotation, sur les deuxièmes moyens de préhension 17.

Lorsque les bouchons 6 et les récipients 2 sont décontaminés, ils sont alors dirigés par le carrousel de traitement 10 vers le carrousel de sortie 11, ce dernier, grâce aux premiers moyens de préhension 13 et aux deuxièmes moyens de préhension 17 qu'il comporte, se saisit alors des bouchons 6 et des récipients 2 décontaminés pour les diriger vers une ligne de convoyage débouchant dans l'unité de bouchage 5.

De préférence, l'unité de bouchage 5 forme également une unité de remplissage pour que les bouchons 6 et les récipients 2 remplis puissent être assemblés.

La mise en place des bouchons 6 entre les cols des récipients 2 permet alors, en une seule étape de décontamination, de décontaminer à la fois les bouchons 6 et les récipients 2, cela au bénéfice de la consommation énergétique de la machine de fabrication 1 puisqu'une seule unité de décontamination 4 est nécessaire, mais également au bénéfice de la compacité de la machine de fabrication 1, pour ces mêmes raisons.

En outre, cela permet de s'assurer qu'un seul circuit stérile soit nécessaire pour convoyer les récipients 2 et les bouchons 6 décontaminés.

## Revendications

1. Procédé de décontamination d'une série de bouchons et d'une série de récipients (2) en matière plastique destinés chacun à être obturés par un des bouchons (6) de la série de bouchons, le procédé comprenant les étapes suivantes :
- émettre un rayonnement de décontamination (15) par les moyens d'émission (14),
- convoyer la série de récipients par des premiers moyens de préhension (13) vers les moyens d'émission (14) selon un chemin de convoyage et
- convoyer la série de bouchons par des deuxièmes moyens de préhension (17) vers les moyens d'émission (14) selon le chemin de convoyage ;
**caractérisée en ce que**, lors du convoyage de la série de bouchons et de la série de récipients, les deuxièmes moyens de préhension (17) sont en alternance le long du chemin de convoyage avec les premiers moyens de préhension (13) devant les moyens d'émission (14).

2. Procédé de décontamination selon la revendication précédente, dans lequel les récipients et bouchons présentent un axe et dans lequel les récipients et/ou les bouchons sont mis en rotation au tour de l'axe au moins pendant l'étape d'émission du rayonnement de décontamination.

3. Procédé de décontamination selon l'une quelconque des revendications précédentes, **caractérisée en ce que** le rayonnement de décontamination comprend un faisceau d'électrons.

4. Unité de décontamination (4) pour la mise en œuvre du procédé selon l'une des revendications précédentes, comprenant :
- des moyens d'émission (14) d'un rayonnement de décontamination (15),
- des premiers moyens de convoyage (12) présentant des premiers moyens de préhension (13), et les convoyer vers les moyens d'émission (14) ;
- des deuxièmes moyens de convoyage (16) présentant des deuxièmes moyens de préhension (17) pour maintenir les bouchons (6), et lesdits premiers moyens de convoyage (12) convoyant lesdits premiers moyens de préhensions (13) vers les moyens d'émission (14),
dans lequel deux premiers moyens de préhension (13) contigus définissent entre eux un espacement (E), **caractérisée en ce que** un des deuxièmes moyens de convoyage (16) est disposés dans l'espacement entre les deux premiers moyens de convoyage (12) contiguës en présentant une portion de convoyage dans laquelle les deuxièmes moyens de préhension (17) sont en alternance avec les premiers moyens de préhension (13) devant les moyens d'émission (14).

5. Unité de décontamination (4) selon la revendication précédente, **caractérisée en ce que** les premiers moyens de préhension (13) et les deuxièmes moyens de préhension (17) sont situés dans un même plan.

6. Unité de décontamination (4) selon l'une quelconque des revendications 4 à 5 **caractérisée en ce qu'**elle comprend :
- une première ligne d'alimentation (7) en récipients ;
- une deuxième ligne d'alimentation (8) en bouchons ;
- un carrousel d'entrée (9) communicant avec la première ligne d'alimentation (7) en récipients (2) et avec la deuxième ligne d'alimentation (8) en bouchons (6) ;
- un carrousel de traitement (10) alimenté par le carrousel d'entrée (9) ;
- un carrousel de sortie (11) alimenté par le carrousel de traitement (10),
chacun du carrousel d'entrée (9), du carrousel de traitement (10) et du carrousel de sortie (11) portant les premiers moyens de convoyage (12) et les deuxièmes moyens de convoyage (16).

7. Unité de décontamination (4) selon la revendication précédente, caractérisée en ce les moyens de préhension (13, 17) comprennent des moyens d'entraînement en rotation (19) des récipients (2) et des bouchons (6) sur le carrousel de traitement (10).

8. Unité de décontamination (4) selon la revendication précédente, **caractérisée en ce que** les moyens d'entraînement en rotation (19) comprennent :
- un tampon (21) destiné à venir au contact des récipients (2) et des bouchons (6) pour les entraîner en rotation par friction ;
- des moyens moteurs (22) pour mettre en rotation les tampons (21).

9. Unité de décontamination (4) selon la revendication précédente **caractérisée en ce que** les tampons (21) sont mobiles entre :
- une position d'utilisation dans laquelle les tampons sont aptes à être en contact des récipients (2) ou des bouchons (6) ;
- une position de repos dans laquelle ils sont écartés des récipients (2) ou des bouchons (6),
chaque tampon (21) étant positionné dans sa position d'utilisation ou dans sa position de repos par un chemin de came (23) et un galet (24) solidaire du tampon (21).

10. Unité de décontamination (4) selon l'une quelconque des revendications 6 à 9, caractérisée en ce les premiers moyens de préhension (13) comprennent des moyens de support (20) des récipients (2) sur le carrousel de traitement (10).

11. Unité de décontamination (4) selon la revendication précédente, **caractérisée en ce que** les moyens de support (20) comprennent des socles (29) destinés à recevoir un fond des récipients (2), chaque socle (29) étant mobile entre :
- une position d'utilisation dans laquelle le socle est apte à être en contact du fond des récipients (2) ;
- une position de repos dans laquelle il est écarté du fond des récipients (2), chaque socle (29) étant positionné dans sa position d'utilisation ou dans sa position de repos par un chemin de came (30) et un galet (31) solidaire du socle (29).

12. Unité de décontamination (4) selon la revendication précédente, **caractérisée en ce que** chaque socle (29) est libre en rotation dans sa position d'utilisation.

13. Unité de décontamination (4) selon l'une quelconque des revendications 4 à 12, **caractérisée en ce que** le premier moyen de convoyage est adapté pour coopérer avec une bague de support du col du récipient.

14. Machine de fabrication (1) de récipients, comprenant :
- une unité de soufflage (3) ;
- une unité de bouchage (5) ;
- une unité de décontamination (4) selon l'une quelconque des revendications 4 à 13,
l'unité de décontamination (4) étant intercalée entre l'unité de soufflage (3) et l'unité de bouchage (5).

## Patentansprüche

1. Verfahren zur Dekontamination einer Reihe von Kappen und einer Reihe von Behältern (2) aus Kunststoff, von denen jeder dazu bestimmt ist, mit einer der Kappen (6) der Reihe von Kappen verschlossen zu werden, wobei das Verfahren die folgenden Schritte umfasst:
- Emittieren einer Dekontaminationsstrahlung (15) durch die Emissionsmittel (14),
- Befördern der Reihe von Behältern durch erste Greifmittel (13) zu den Emissionsmitteln (14) gemäß einem Förderweg und
- Befördern der Reihe von Kappen durch zweite Greifmittel (17) zu den Emissionsmitteln (14) gemäß einem Förderweg;
**dadurch gekennzeichnet, dass**, beim Befördern der Reihe von Kappen und der Reihe von Behältern, die zweiten Greifmittel (17) entlang des Förderwegs mit den ersten Greifmitteln (13) vor den Emissionsmitteln (14) alternieren.

2. Verfahren zur Dekontamination nach dem vorhergehenden Anspruch, wobei die Behälter und Kappen eine Achse aufweisen und wobei die Behälter und/oder die Kappen mindestens während des Schritts des Emittierens der Dekontaminationsstrahlung um die Achse herum in Drehung versetzt werden.

3. Verfahren zur Dekontamination nach einem der vorhergehenden Ansprüche, **dadurch gekennzeichnet, dass** die Dekontaminationsstrahlung einen Elektronenstrahl umfasst.

4. Dekontaminationseinheit (4) zur Implementierung des Verfahrens nach einem der vorhergehenden Ansprüche, umfassend:
- Emissionsmittel (14) zum Emittieren einer Dekontaminationsstrahlung (15),
- erste Fördermittel (12), die erste Greifmittel (13) aufweisen, und sie zu den Emissionsmitteln (14) zu befördern;
- zweite Fördermittel (16), die zweite Greifmittel (17) aufweisen, um die Kappen (6) zu halten, und wobei die ersten Fördermittel (12) die ersten Greifmittel (13) zu den Emissionsmitteln (14) befördern,
wobei zwei aneinandergrenzende erste Greifmittel (13) zwischen sich einen Abstand (E) definieren, **dadurch gekennzeichnet, dass** eines der zweiten Fördermittel (16) in dem Abstand zwischen den beiden aneinandergrenzenden ersten Fördermitteln (12) angeordnet ist, wobei es einen Förderabschnitt aufweist, in dem die zweiten Greifmittel (17) mit den ersten Greifmitteln (13) vor den Emissionsmitteln (14) alternieren.

5. Dekontaminationseinheit (4) nach dem vorhergehenden Anspruch, **dadurch gekennzeichnet, dass** die ersten Greifmittel (13) und die zweiten Greifmittel (17) in einer selben Ebene gelegen sind.

6. Dekontaminationseinheit (4) nach einem der Ansprüche 4 bis 5, **dadurch gekennzeichnet, dass** sie umfasst:
- eine erste Beschickungslinie (7) zur Beschickung mit Behältern;
- eine zweite Beschickungslinie (8) zur Beschickung mit Kappen;
- ein Eingangskarussell (9), das mit der ersten Beschickungslinie (7) zur Beschickung mit Behältern (2) und mit der zweiten Beschickungslinie (8) zur Beschickung mit Kappen (6) in Verbindung steht;
- ein Behandlungskarussell (10), das von dem Eingangskarussell (9) beschickt wird;
- ein Ausgangskarussell (11), das von dem Behandlungskarussell (10) beschickt wird,
wobei unter dem Eingangskarussell (9), dem Behandlungskarussell (10) und dem Ausgangskarussell (11) jedes die ersten Fördermittel (12) und die zweiten Fördermittel (16) trägt.

7. Dekontaminationseinheit (4) nach dem vorhergehenden Anspruch, **dadurch gekennzeichnet, dass** die Greifmittel (13, 17) Drehantriebsmittel (19) für die Behälter (2) und die Kappen (6) auf dem Behandlungskarussell (10) umfassen.

8. Dekontaminationseinheit (4) nach dem vorhergehenden Anspruch, **dadurch gekennzeichnet, dass** die Drehantriebsmittel (19) umfassen:
- einen Puffer (21), der dazu bestimmt ist, mit den Behältern (2) und den Kappen (6) in Kontakt zu gelangen, um sie durch Reibung drehanzutreiben;
- Motormittel (22), um die Puffer (21) in Drehung zu versetzen.

9. Dekontaminationseinheit (4) nach dem vorhergehenden Anspruch, **dadurch gekennzeichnet, dass** die Puffer (21) beweglich sind zwischen:
- einer Verwendungsstellung, in der die Puffer geeignet sind, mit den Behältern (2) oder den Kappen (6) in Kontakt zu sein;
- einer Ruhestellung, in der sie von den Behältern (2) oder den Kappen (6) beabstandet sind,
wobei jeder Puffer (21) durch eine Kulisse (23) und eine Rolle (24), die fest mit dem Puffer (21) verbunden ist, in seine Verwendungsstellung oder in seine Ruhestellung positioniert wird.

10. Dekontaminationseinheit (4) nach einem der Ansprüche 6 bis 9, **dadurch gekennzeichnet, dass** die ersten Greifmittel (13) Stützmittel (20) für die Behälter (2) auf dem Behandlungskarussell (10) umfassen.

11. Dekontaminationseinheit (4) nach dem vorhergehenden Anspruch, **dadurch gekennzeichnet, dass** die Stützmittel (20) Sockel (29) umfassen, die dazu bestimmt sind, einen Boden der Behälter (2) aufzunehmen, wobei jeder Sockel (29) beweglich ist zwischen:
- einer Verwendungsstellung, in welcher der Sockel geeignet ist, mit dem Boden der Behälter (2) in Kontakt zu sein;
- einer Ruhestellung, in der er von dem Boden der Behälter (2) beabstandet ist, wobei jeder Sockel (29) durch eine Kulisse (30) und eine Rolle (31), die fest mit dem Sockel (29) verbunden ist, in seine Verwendungsstellung oder in seine Ruhestellung positioniert wird.

12. Dekontaminationseinheit (4) nach dem vorhergehenden Anspruch, **dadurch gekennzeichnet, dass** jeder Sockel (29) in seiner Verwendungsstellung drehbeweglich ist.

13. Dekontaminationseinheit (4) nach einem der Ansprüche 4 bis 12, **dadurch gekennzeichnet, dass** das erste Fördermittel dazu angepasst ist, mit einem Stützring für den Hals des Behälters zusammenzuwirken.

14. Maschine (1) zur Herstellung von Behältern, umfassend:
- eine Blaseinheit (3);
- eine Verschließeinheit (5);
- eine Dekontaminationseinheit (4) nach einem der Ansprüche 4 bis 13,
wobei die Dekontaminationseinheit (4) zwischen der Blaseinheit (3) und der Verschließeinheit (5) eingefügt ist.

## Claims

1. Method for decontaminating a series of caps and a series of containers (2) made of plastics material, each intended to be closed by one of the caps (6) of the series of caps, the method comprising the following steps:
- emitting decontaminating radiation (15) using emission means (14),
- conveying the series of containers using first gripping means (13) towards the emission means (14) along a conveying path, and
- conveying the series of caps using second gripping means (17) towards the emission means (14) along the conveying path;
**characterized in that**, during the conveying of the series of caps and of the series of containers, the second gripping means (17) alternate along the conveying path with the first gripping means (13) in front of the emission means (14).

2. Decontamination method according to the preceding claim, wherein the containers and caps have an axis and wherein the containers and/or the caps are rotated about the axis at least during the step of emitting the decontaminating radiation.

3. Decontamination method according to any one of the preceding claims, **characterized in that** the decontaminating radiation comprises an electron beam.

4. Decontamination unit (4) for implementing the method according to one of the preceding claims, comprising:
- means (14) for emitting decontaminating radiation (15),
- first conveying means (12) having first gripping means (13), and conveying them to the emission means (14);
- second conveying means (16) having second gripping means (17) for holding the caps (6), and said first conveying means (12) conveying said first gripping means (13) to the emission means (14),
wherein two contiguous first gripping means (13) define a space (E) between them, **characterized in that** one of the second conveying means (16) is arranged in the space between the two contiguous first conveying means (12), creating a conveying portion in which the second gripping means (17) alternate with the first gripping means (13) in front of the emission means (14).

5. Decontamination unit (4) according to the preceding claim, **characterized in that** the first gripping means (13) and the second gripping means (17) are situated in one and the same plane.

6. Decontamination unit (4) according to either one of Claims 4 and 5, **characterized in that** it comprises:
- a first line (7) for supplying containers;
- a second line (8) for supplying caps;
- an input carousel (9) in communication with the first line (7) for supplying containers (2) and with the second line (8) for supplying caps (6);
- a treatment carousel (10) supplied by the input carousel (9);
- an output carousel (11) supplied by the treatment carousel (10),
each of the input carousel (9), the treatment carousel (10) and the output carousel (11) bearing the first conveying means (12) and the second conveying means (16).

7. Decontamination unit (4) according to the preceding claim, **characterized in that** the gripping means (13, 17) comprise means (19) for driving the containers (2) and the caps (6) in rotation on the treatment carousel (10).

8. Decontamination unit (4) according to the preceding claim, **characterized in that** the rotational drive means (19) comprise:
- a pad (21) intended to come into contact with the containers (2) and the caps (6) in order to drive them in rotation by friction;
- drive means (22) for rotating the pads (21).

9. Decontamination unit (4) according to the preceding claim, **characterized in that** the pads (21) are moveable between:
- a usage position in which the pads can be in contact with the containers (2) or the caps (6);
- an idle position in which they are spaced apart from the containers (2) or the caps (6),
each pad (21) being positioned in its usage position or in its idle position by a cam track (23) and a roller (24) secured to the pad (21).

10. Decontamination unit (4) according to any one of Claims 6 to 9, **characterized in that** the first gripping means (13) comprise means (20) for supporting the containers (2) on the treatment carousel (10).

11. Decontamination unit (4) according to the preceding claim, **characterized in that** the support means (20) comprise bases (29) intended to receive a bottom of the containers (2), each base (29) being moveable between:
- a usage position in which the base can be in contact with the bottom of the containers (2);
- an idle position in which it is spaced apart from the bottom of the containers (2), each base (29) being positioned in its usage position or in its idle position by a cam track (30) and a roller (31) secured to the base (29).

12. Decontamination unit (4) according to the preceding claim, **characterized in that** each base (29) is free to rotate in its usage position.

13. Decontamination unit (4) according to any one of Claims 4 to 12, **characterized in that** the first conveying means is designed to cooperate with a support ring for the neck of the container.

14. Machine (1) for manufacturing containers, comprising:
- a blow moulding unit (3);
- a capping unit (5);
- a decontamination unit (4) according to any one of Claims 4 to 13,
the decontamination unit (4) being interposed between the blow moulding unit (3) and the capping unit (5).
